# EUROPEAN PATENT APPLICATION

(11) **EP 4 736 753 A1**
(43) Date of publication of application: **06.05.2026**
(21) Application number: 24864359.5
(22) Date of filing: 13.08.2024
(51) Int. Cl.: A61B 5/0225

(54) **BLOOD PRESSURE MEASUREMENT METHOD AND SYSTEM, ELECTRONIC DEVICE, AND MEDIUM**

(30) Priority: 15.09.2023 CN 202311199863
(71) Applicant: Huawei Technologies Co., Ltd., Shenzhen, Guangdong 518129 (CN)
(72) Inventor: LI, Weinan, Shenzhen, Guangdong 518129 (CN); JIN, Junye, Shenzhen, Guangdong 518129 (CN); WANG, Youhua, Shenzhen, Guangdong 518129 (CN)
(74) Representative: Grünecker Patent- und Rechtsanwälte PartG mbB
(86) International application number: PCT/CN2024/111717
(87) International publication number: WO 2025/055636

(57) **Abstract**

This application relates to the field of blood pressure measurement, and discloses a blood pressure measurement method and system, an electronic device, and a medium. According to the blood pressure measurement method in this application, in a pressurization phase of blood pressure measurement, a real-time air pressure value of an air path connected to an air bladder is detected, and then an air valve drive circuit is controlled based on the real-time air pressure value. For example, when air pressure of the air bladder is low, a low drive voltage may be used to drive an air valve to operate; or when the air pressure of the air bladder is high, a high drive voltage may be used to drive the air valve to operate. In this case, compared with a manner of using a fixed high drive voltage to drive the air valve to operate, dynamically adjusting a drive voltage of the air valve in the pressurization process allows the air valve to complete operation with low power consumption while meeting a deflation amount requirement. As a result, overall power consumption of the blood pressure measurement system can be reduced by optimizing power consumption in each blood pressure measurement process, thereby prolonging a battery life of a wearable device and finally implementing long-term blood pressure monitoring.

## Description

This application claims priority to Chinese Patent Application No. 202311199863.1, filed with the China National Intellectual Property Administration on September 15, 2023 and entitled "BLOOD PRESSURE MEASUREMENT METHOD AND SYSTEM, ELECTRONIC DEVICE, AND MEDIUM", which is incorporated herein by reference in its entirety.

### TECHNICAL FIELD

This application relates to the field of blood pressure measurement technologies, and in particular, to a blood pressure measurement method and system, an electronic device, and a medium.

### BACKGROUND

Currently, with the continuous development of technologies, wearable devices have become a part of people's life. People are no longer satisfied with simple monitoring of time and step count, but expect to use the wearable devices to detect their physical health indicators. For example, blood pressure, as a fundamental physiological indicator of a human body, is an important basis for assessing a plurality of diseases. Therefore, the wearable devices are generally integrated with blood pressure measurement systems. Different from a conventional cuff-type blood pressure monitor, a wearable blood pressure measurement device has higher requirements for both size and power consumption.

Although an existing wearable blood pressure measurement device, such as a wrist-type blood pressure measurement device, can achieve a small size, a battery capacity and a battery life are significantly constrained, making it difficult to implement long-term continuous monitoring. Therefore, it is necessary to reduce power consumption of the wearable blood pressure measurement device to implement a long battery life or even an ultra-long battery life.

### SUMMARY

Embodiments of this application provide a blood pressure measurement method and system, an electronic device, and a medium.

According to a first aspect, an embodiment of this application provides a blood pressure measurement method, applied to an electronic device. The electronic device includes an air bladder and an air valve that are configured for blood pressure measurement. The method includes: When the electronic device enters a pressurization phase of blood pressure measurement, the electronic device obtains a real-time air pressure value in the air bladder; determines a real-time drive voltage of the air valve based on the real-time air pressure value and a first deflation condition; and drives, based on the real-time drive voltage, the air valve to operate.

It may be understood that in this application, in a pressurization phase of blood pressure measurement, a sensor detects the real-time air pressure value in the air bladder, and then an air valve drive circuit is controlled based on the real-time air pressure value, so that a real-time drive voltage applied by the air valve drive circuit to the air valve corresponds to the real-time air pressure value. For example, when air pressure of the air bladder is low, a low drive voltage may be used to drive the air valve to operate, or when the air pressure of the air bladder is high, a high drive voltage may be used to drive the air valve to operate. The real-time drive voltage may be a minimum drive voltage that allows the air valve to meet the first deflation condition (for example, a deflation amount is 0). In this way, a drive voltage of the air valve is dynamically adjusted in the pressurization process, so that the air valve can complete operation with low power consumption while still meeting the first deflation condition. In this way, a battery life of the electronic device can be prolonged by optimizing power consumption in each blood pressure measurement process, thereby implementing long-term blood pressure monitoring.

In a possible implementation of the first aspect, determining the real-time drive voltage of the air valve based on the real-time air pressure value and the first deflation condition includes: when the first deflation condition is that a deflation amount of the air valve is less than or equal to a first deflation amount, obtaining a correspondence between a voltage of the air valve and air pressure of the air bladder; determining, based on the correspondence between the voltage of the air valve and the air pressure of the air bladder, a voltage of the air valve corresponding to the real-time air pressure value; and determining the voltage of the air valve corresponding to the real-time air pressure value as the real-time drive voltage of the air valve.

The first deflation amount may be 0, or a value infinitely close to 0. That the deflation amount of the air valve is less than or equal to 0 may be understood as that the air valve does not release air. When the air valve is required not to release air, the correspondence between the voltage of the air valve and the air pressure of the air bladder is obtained. The correspondence between the voltage of the air valve and the air pressure of the air bladder indicates voltages of the air valve corresponding to different air pressure of the air bladder. The voltage of the air valve is a minimum voltage that ensures that the air valve does not release air. Therefore, the voltage of the air valve corresponding to the real-time air pressure value may be directly determined from the correspondence between the voltage of the air valve and the air pressure of the air bladder, and then the voltage of the air valve is determined as the real-time drive voltage of the air valve. In this way, in the pressurization phase of the blood pressure measurement, it can be ensured that the air valve does not release air, and the air valve completes operation with a small power, thereby reducing power consumption in the blood pressure measurement process.

In a possible implementation of the first aspect, in the correspondence between the voltage of the air valve and the air pressure of the air bladder, as the air pressure of the air bladder increases, the voltage of the air valve exhibits a trend of first decreasing and then increasing.

It is considered that when the air pressure of the air bladder is low, the air valve needs only a small magnetic attraction to block a deflation hole to ensure that a system does not release air. When the air pressure of the air bladder is high, the air valve only needs a large magnetic attraction to block the deflation hole to ensure that the system does not release air. Therefore, in the correspondence between the voltage of the air valve and the air pressure of the air bladder, as the air pressure of the air bladder increases, the voltage of the air valve decreases first and then increases. In this way, when the drive voltage of the air valve is dynamically adjusted, the drive voltage of the air valve first decreases and then increases, thereby reducing a power loss of the air valve at an initial inflation stage, and reducing overall power consumption of a blood pressure measurement system.

Actually, when the correspondence between the voltage of the air valve and the air pressure of the air bladder is calibrated, a relationship curve, between the voltage of the air valve and the air pressure of the air bladder, representing the correspondence between the voltage of the air valve and the air pressure of the air bladder may be obtained through curve fitting.

In a possible implementation of the first aspect, determining the real-time drive voltage of the air valve based on the real-time air pressure value and the first deflation condition includes: when the first deflation condition is that a deflation amount of the air valve is greater than a second deflation amount, obtaining a real-time ideal air pressure value of the air bladder, where the real-time ideal air pressure value represents a corresponding air pressure value at which the air bladder meets a linear pressurization condition; and determining the real-time drive voltage of the air valve based on a real-time deviation value between the real-time ideal air pressure value and the real-time air pressure value.

It may be understood that linear voltage pressurization of the air bladder helps improve accuracy of blood pressure measurement. In this application, the real-time drive voltage of the air valve is controlled, to control the air valve to release a corresponding deflation amount, so that the air bladder meets the linear pressurization condition. Therefore, the second deflation amount may be 0, or a value infinitely close to 0. That the deflation amount of the air valve is greater than 0 may be understood as that the deflation hole of the air valve is open and the air valve releases air. The deflation amount of the air valve is determined by the drive voltage of the air valve. A higher drive voltage of the air valve indicates a smaller deflation amount, and a lower drive voltage of the air valve indicates a larger deflation amount. When the deflation hole of the air valve is open, an actual deflation amount of the air valve needs to allow the air bladder to meet the linear pressurization condition. Therefore, the drive voltage of the air valve is controlled by using the deviation value between the real-time ideal air pressure value at which the air bladder meets the linear pressurization and the real-time air pressure value, so that under a corresponding real-time drive voltage, it is ensured that the air valve completes operation with low power consumption, and it is also ensured that the air bladder performs linear pressurization, thereby helping improve accuracy of blood pressure measurement.

In a possible implementation of the first aspect, the electronic device further includes an air pump configured for blood pressure measurement. Determining the real-time drive voltage of the air valve based on the real-time deviation value between the real-time ideal air pressure value and the real-time air pressure value includes: determining a real-time drive voltage of the air pump and the real-time drive voltage of the air valve based on the real-time deviation value by using a proportional-integral-derivative control algorithm.

It may be understood that the air valve is configured to deflate the air bladder, and the air pump is mainly configured to inflate the air bladder. In the pressurization phase, the air bladder is inflated by using the air pump while being deflated by using the air valve. As described above, the air bladder may be linearly pressurized by controlling the deflation amount of the air valve. During actual application, the air bladder may alternatively be linearly pressurized by controlling both the deflation amount of the air valve and the drive voltage of the air pump. In this way, the air pump can operate in an optimal operating state while ensuring the linear pressurization of the air bladder, thereby further improving power of the air pump and improving inflating efficiency of blood pressure measurement.

In a possible implementation of the first aspect, the electronic device further includes a first drive circuit configured to drive the air pump to operate. The method further includes: controlling, through a variable power supply control chip in the first drive circuit, the drive voltage of the air pump to be the real-time drive voltage of the air pump.

It may be understood that the first drive circuit is configured to drive the air pump to operate. In the pressurization phase, the variable power supply control chip in the first drive circuit may dynamically adjust a drive voltage applied to the air pump, so that the air pump is in the optimal operating state, thereby improving inflating efficiency.

In a possible implementation of the first aspect, obtaining the real-time ideal air pressure value of the air bladder includes: obtaining a linear pressurization curve, where the linear pressurization curve includes ideal air pressure values corresponding to different moments; and determining, based on the linear pressurization curve, an ideal air pressure value corresponding to a current moment, to obtain the real-time ideal air pressure value.

In a possible implementation of the first aspect, the electronic device further includes a sensor connected to the air bladder through an air path. Obtaining the real-time air pressure value in the air bladder includes: detecting air pressure in the air bladder by using the sensor, to obtain the real-time air pressure value.

It may be understood that, to obtain an accurate real-time air pressure value in time to dynamically adjust the voltage of the air valve in the pressurization phase, the sensor may send a detected air pressure value to a processor in real time.

The sensor may be a differential pressure sensor. The differential pressure sensor may directly detect a difference between air pressure inside the air bladder and air pressure outside the air bladder (that is, atmospheric pressure). The difference may more intuitively reflect an expansion degree of the air bladder.

In a possible implementation of the first aspect, the electronic device further includes a second drive circuit configured to drive the air valve to operate. Driving, based on the real-time drive voltage, the air valve to operate includes: controlling, through a variable power supply control chip in the second drive circuit, a drive voltage of the air valve to be the real-time drive voltage, so that the deflation amount of the air valve meets the first deflation condition.

It may be understood that the second drive circuit is configured to drive the air valve to operate. In the pressurization phase, the variable power supply control chip in the second drive circuit may dynamically adjust a drive voltage applied to the air valve, so that the air valve achieves no air release with low power consumption, or the air valve releases air based on a required deflation amount.

According to a second aspect, an embodiment of this application provides a blood pressure measurement system, including an air bladder, an air valve, and a blood pressure measurement apparatus. The blood pressure measurement apparatus is configured to: in a pressurization phase of blood pressure measurement, obtain a real-time air pressure value in the air bladder; determine a real-time drive voltage of the air valve based on the real-time air pressure value and a first deflation condition; and drive, based on the real-time drive voltage, the air valve to operate.

According to a third aspect, an embodiment of this application provides a readable medium. The readable medium stores instructions, and when the instructions are executed on an electronic device, the electronic device is enabled to perform the blood pressure measurement method in the first aspect.

According to a fourth aspect, an embodiment of this application provides an electronic device. The electronic device includes: a memory, configured to store instructions executed by one or more processors of the electronic device; and a processor, where the processor is one of the processors of the electronic device and is configured to perform the blood pressure measurement method in the first aspect.

### BRIEF DESCRIPTION OF DRAWINGS

FIG. 1 is a diagram of a wristband device according to an embodiment of this application;
FIG. 2 is a diagram of a first relationship curve between air pressure of an air bladder and a voltage of an air valve according to an embodiment of this application;
FIG. 3 is a schematic flowchart of a first blood pressure measurement method according to an embodiment of this application;
FIG. 4 is a diagram of a drive voltage control curve according to an embodiment of this application;
FIG. 5 is a schematic flowchart of a second blood pressure measurement method according to an embodiment of this application;
FIG. 6 is a diagram of a second relationship curve between air pressure of an air bladder and a voltage of an air valve according to an embodiment of this application;
FIG. 7 is a diagram of a blood pressure measurement control process according to an embodiment of this application;
FIG. 8 is a schematic flowchart of a third blood pressure measurement method according to an embodiment of this application;
FIG. 9 is a flowchart of a blood pressure measurement control process according to an embodiment of this application;
FIG. 10 is a diagram of a PID control process according to an embodiment of this application;
FIG. 11 is a schematic flowchart of a PID control manner according to an embodiment of this application; and
FIG. 12 is a diagram of trends of a fourth drive voltage and a fifth drive voltage that change with air pressure of the air bladder according to an embodiment of this application.

### DESCRIPTION OF EMBODIMENTS

Various aspects of illustrative embodiments are described below by using terms commonly used by a person skilled in the art.

In general, a wearable device usually measure blood pressure by using an oscillometric method. For example, a blood pressure measurement system in the wearable device usually includes an air bladder, an air pump configured to inflate the air bladder, and an air valve configured to deflate the air bladder.

In some embodiments, a blood pressure measurement process is as follows: The air bladder or the like is bound to a to-be-measured part of a person, for example, a wrist. In a pressurization phase of measurement, the air pump is opened, and the air valve is usually closed to ensure air tightness. For example, a preset high fixed drive voltage is used to drive the air valve to operate, so that the air valve enhances a magnetic attraction to block a deflation hole. Then, the air pump inflates the air bladder to pressurize the air bladder, thereby blocking an arterial blood flow of a human body. In a depressurization phase of measurement, the air valve is opened, and gas in the air bladder is slowly released through the air valve, or gas is released through both the air pump and the air valve, so that a pulse wave signal of the human body is transmitted to an air chamber of the air bladder, and pressure fluctuation occurs in the air chamber of the air bladder. Finally, blood pressure of the user may be obtained by analyzing an air pressure fluctuation status in the air chamber of the air bladder.

A person wants to implement long-term blood pressure monitoring by wearing the wearable device. However, due to a limited size of the wearable device, it is difficult to equip the wearable device with a large-capacity battery currently, and therefore a battery life is poor. Therefore, how to improve the battery life of the wearable device to implement long-term blood pressure monitoring by using the blood pressure measurement system in the wearable device is an urgent problem to be resolved.

To improve the battery life of the wearable device, in an implementation, a kernel operating mode and a resource allocation manner of a processor in the wearable device are controlled. For example, the processor enters a standby mode during a blood pressure measurement interval, so that the processor is in a low power consumption state. Moreover, the blood pressure measurement interval is increased, that is, a blood pressure measurement frequency is reduced, so that the processor is in the low power consumption state for a longer time, thereby reducing power consumption of the device. However, only power consumption of the processor can be reduced by controlling an operating manner of the processor. Actually, the processor is not the only device that consumes power, and a device such as an air pump or an air valve also consumes power. Therefore, optimization of overall power consumption in this manner is very limited, and reducing the blood pressure measurement frequency is not conducive to timely detecting an abnormal blood pressure problem.

Therefore, an embodiment of this application provides a blood pressure measurement method, applied to a blood pressure measurement system in a wearable device. Specifically, when air pressure of the air bladder is low, an air valve needs only a small magnetic attraction to block a deflation hole to ensure that the system does not release air. When the air pressure of the air bladder is high, the air valve only needs a large magnetic attraction to block the deflation hole to ensure that the system does not release air. Therefore, in this method, in a pressurization phase of blood pressure measurement, a sensor detects a real-time air pressure value of an air path connected to an air bladder, and then an air valve drive circuit is controlled based on the real-time air pressure value, so that a real-time drive voltage applied by the air valve drive circuit to the air valve corresponds to the real-time air pressure value of the air path connected to the air bladder. For example, when the air pressure of the air bladder is low, a low drive voltage may be used to drive the air valve to operate, or when the air pressure of the air bladder is high, a high drive voltage may be used to drive the air valve to operate. The real-time drive voltage may be a minimum voltage that meets a deflation amount requirement of the blood pressure measurement system under real-time air pressure (for example, a deflation amount is 0, that is, there is no deflation or the deflation amount is less than a preset threshold). In this case, compared with a manner of using a fixed high drive voltage to drive the air valve to operate, dynamically adjusting a drive voltage of the air valve in the pressurization process allows the air valve to complete operation with low power consumption while meeting the deflation amount requirement. As a result, without affecting a blood pressure measurement frequency, overall power consumption of the blood pressure measurement system can be reduced by optimizing power consumption in each blood pressure measurement process, thereby prolonging a battery life of the wearable device and finally implementing long-term blood pressure monitoring.

The following further describes implementations of this application in detail with reference to the accompanying drawings.

First, the wearable device mentioned in this application may include any one of a wristband device (for example, a smartwatch or a smart band), a cuff-type device, a head mounted device, or a finger mounted device, depending on different wearing or mounting positions. The blood pressure measurement method in this application may be applied to any wearable device with a blood pressure measurement function. For ease of description, the following uses an example in which the wearable device is a wristband device for description.

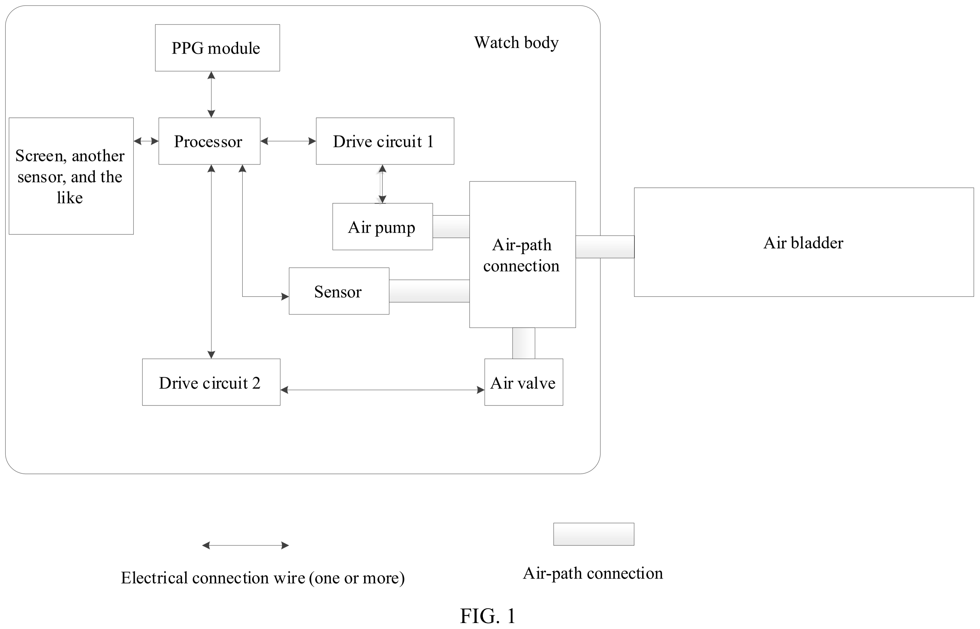

FIG. 1 is a diagram of a wristband device 100 according to an embodiment of this application. A blood pressure measurement system may be integrated into the wristband device 100, so that the wristband device 100 has a blood pressure measurement function.

As shown in FIG. 1, the wristband device 100 may include a watch body, and the watch body is configured to implement a main function of the wristband device 100, for example, display, audio playing, communication, and blood pressure measurement. The blood pressure measurement system may include a processor, a drive circuit 1, an air pump, a sensor, a drive circuit 2, an air valve, and a PPG module that are integrated into the watch body, and an air bladder connected to the watch body. The following describes devices in the blood pressure measurement system in detail. Details are not described herein. It should be noted that a manner of disposing the blood pressure measurement system in the wearable device is not limited in this application. For example, the air pump, the sensor, and the air valve may alternatively be disposed outside the watch body based on an actual requirement. Specifically, the sensor may be disposed inside the air bladder. An advantage of integrating some devices in the blood pressure measurement system into the watch body is that comfort of a user can be improved.

Refer to FIG. 1. The sensor, the air pump, the air valve, and the air bladder are connected through the air path. The sensor detects an air pressure value in the air path connected to the air bladder, so that real-time air pressure in the air bladder can be learned in time in a blood pressure measurement process. In an implementation, the sensor may be a differential pressure sensor. The differential pressure sensor may directly detect a difference between air pressure inside the air bladder and air pressure outside the air bladder (that is, atmospheric pressure). The difference may more intuitively reflect an expansion degree of the air bladder. Therefore, the real-time air pressure in the air bladder mentioned in this application may be understood as a difference between the air pressure in the air bladder and the atmospheric pressure.

The drive circuit 1 is electrically connected to the air pump, and is configured to control an operating status of the air pump. The drive circuit 1 includes a necessary key chip, such as a power supply control chip configured to control a drive voltage of the air pump, and a peripheral resistance-capacitance circuit that protects the chip, to ensure that the chip in a normal operating range is not damaged due to an excessive current impact. In an implementation, the power supply control chip may be a variable power supply control chip. The variable power supply control chip may adjust the drive voltage of the air pump, so that the air pump is in a desired operating state. For example, increasing the drive voltage of the air pump may allow the air pump to increase an inflating rate of the air bladder. By comparison, an invariable power supply control chip can drive the air pump to operate only by a fixed drive voltage, which is not conducive to adjusting the inflating rate of the air bladder. Because control of the inflating rate is a key to improving accuracy of blood pressure measurement, the drive circuit 1 controls the air pump through the variable power supply control chip, thereby helping improve accuracy of blood pressure measurement.

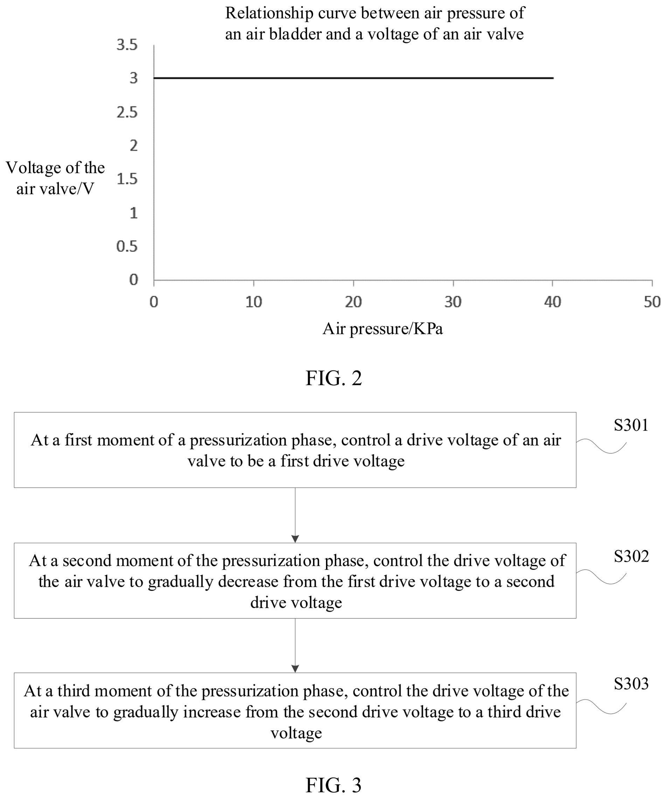

The drive circuit 2 is electrically connected to the air valve, and is configured to control an operating status of the air valve. Similar to the drive circuit 1, the drive circuit 2 also includes a necessary key chip, such as a power supply control chip configured to control a drive voltage of the air valve, and a peripheral resistance-capacitance circuit. In an implementation, the power supply control chip may be an invariable power supply control chip, and the invariable power supply control chip drives the air valve to operate by using a fixed drive voltage. Usually, when the blood pressure measurement starts, to ensure air tightness, a high fixed drive voltage is used to drive the air valve to operate, so that the air valve enhances a magnetic attraction to block a deflation hole. However, because the invariable power supply control chip can drive the air valve only by using the fixed drive voltage, the drive voltage of the air valve remains high throughout the entire pressurization phase, that is, in a process in which air pressure of the air bladder slowly increases. For example, FIG. 2 is a diagram of a relationship curve between air pressure of an air bladder and a voltage of an air valve according to an embodiment of this application. As shown in FIG. 2, the voltage of the air valve, that is, the drive voltage of the air valve, is fixed. However, when the air pressure of the air bladder is low, the air valve only needs a small magnetic attraction to block the deflation hole to ensure that the system does not release air. The drive voltage of the air valve is excessively high, causing a waste of power consumption. Therefore, in another implementation, the power supply control chip may be a variable power supply control chip. The variable power supply control chip is used, so that when the air pressure of the air bladder is low, the air valve is driven with a minimum drive voltage while still meeting a deflation requirement of the system. In this way, power consumption of the air valve can be reduced.

The PPG module is a functional module that detects health data such as a heart rate, blood oxygen, and blood pressure by using a photoplethysmography (photoplethysmography, PPG) method. A PPG detection principle is as follows: Because a change of light absorption by skin is directly related to a change of an arterial blood flow velocity, the change of the arterial blood flow velocity may be deduced as long as the change of light absorption by skin is measured, and further a blood pressure value at a specific time point may be obtained. Usually, the PPG module includes an optical transmitter (for example, a light-emitting diode), an optical sensor (for example, a photodiode), an analog front-end chip, and an accelerometer. A PPG detection process is as follows: The optical transmitter illuminates a light wave into the skin, the optical sensor receives variations in light intensity, and finally the analog front-end chip converts an analog signal into a digital signal to obtain an original PPG signal. The accelerometer may measure a motion status of a human body. The motion status of the human body and an optical signal may be used together as an input of a PPG algorithm. In a later stage of PPG detection, an original signal from the analog front-end chip and the accelerometer may be processed by using a complex algorithm, to generate other biological measurement data such as a heart rate, blood oxygen, and blood pressure. It may be understood that the blood pressure measurement system shown in FIG. 1 may measure blood pressure in two different manners: a manner based on the photoplethysmography method by using the PPG module, and a manner based on an oscillometric method by using the drive circuit 1, the drive circuit 2, the air pump, the air valve, the air bladder, and the sensor. The PPG module can implement air bladder-free, non-compression blood pressure measurement, but software and hardware costs are high, and an application scope is smaller compared to the oscillometric method. Therefore, a blood pressure measurement system mentioned below mainly refers to a system that implements blood pressure measurement by using the oscillometric method.

The processor is electrically connected to the drive circuit 1, the sensor, the drive circuit 2, and the PPG module. Herein, the processor may be a processor configured to implement the main function in the wristband device 100. The processor may receive data sent by any module in the drive circuit 1, the sensor, the drive circuit 2, and the PPG module, perform corresponding processing on the data, and send a related instruction to any module. For example, the processor obtains a real-time air pressure value detected by the sensor (for example, a differential pressure sensor), determines, based on the real-time air pressure value, a minimum drive voltage at which the air valve meets the deflation requirement of the system (for example, no deflation or the deflation amount is 0), and then sends a corresponding instruction to the drive circuit 2, to instruct the drive circuit 2 to drive the air valve to operate with the minimum drive voltage. For another example, the processor may alternatively send an air pump control instruction to the drive circuit 1, to instruct the drive circuit 1 to control the operating status of the air pump.

The wristband device 100 shown in FIG. 1 may further include a wristband (not shown in the figure). One end of the air bladder may be connected to the watch body, and the other end of the air bladder is connected to the wristband. In this way, during measurement, the air bladder and the wristband may be wound around the wrist of the user. In an implementation, the air bladder is detachably connected to the watch body. In this way, the air bladder may be flexibly assembled and disassembled based on an actual user requirement.

It should be noted that the blood pressure measurement system shown in FIG. 1 includes the drive circuit 1 and the drive circuit 2, which are respectively configured to control the air pump and the air valve. During actual application, a quantity of drive circuits may be designed based on a specific structure of the system. For example, when the system includes a plurality of air pumps or air valves, a corresponding drive circuit may be disposed for each air valve and each air pump. A specific structure of the blood pressure measurement system is not limited in this application.

The following describes a specific implementation of a blood pressure measurement method in this application based on the description of the wristband device shown in FIG. 1. The blood pressure measurement method in this application may be performed by the processor in the wristband device.

According to the blood pressure measurement method in this application, the drive voltage of the air valve may be dynamically adjusted in the pressurization process, so that the air valve may complete operation with low power consumption. In this way, overall power consumption of the blood pressure measurement system may be reduced by optimizing power consumption in each blood pressure measurement process, thereby prolonging a battery life of the wearable device, and implementing long-term blood pressure monitoring.

This application provides three specific implementations of the blood pressure measurement method. The following describes each specific implementation in detail.

First, a first implementation of the blood pressure measurement method is described. In the first implementation, the processor controls, at different moments in the pressurization process, different drive voltages are used to drive the air valve to operate, to dynamically adjust the drive voltage of the air valve, thereby ensuring that the air valve can complete operation with low power consumption.

FIG. 3 is a schematic flowchart of a first blood pressure measurement method according to an embodiment of this application. As shown in FIG. 3, the blood pressure measurement method may include the following steps.

S301: At a first moment of a pressurization phase, control a drive voltage of the air valve to be a first drive voltage.

The first moment of the pressurization phase may be a start moment of blood pressure measurement, that is, a moment at which the air bladder starts to be inflated. In this case, the drive voltage of the air valve is controlled to be the first drive voltage, and the first drive voltage needs to ensure that the air valve does not release air. For example, the first drive voltage may be a maximum voltage in a drive voltage range that ensures normal operation of the air valve. It may be understood that, when inflation begins, the air valve is powered on at a maximum voltage, to ensure that a deflation path is closed and ensure air tightness.

As mentioned above, in an implementation, a voltage of the air valve, that is, the drive voltage of the air valve, is fixed in the entire pressurization phase, and a related relationship curve between the voltage of the air valve and air pressure of the air bladder is shown in FIG. 2. However, when the air pressure of the air bladder is low, for example, lower than 5 KPa, the air valve needs only a small magnetic attraction to block the deflation hole, and a possibility of deflation is low. If the voltage of the air valve remains high (for example, 3 V), power consumption may be wasted.

Based on this, in an implementation, at the first moment of the pressurization phase, the drive voltage of the air valve is controlled to be the first drive voltage, and drive duration corresponding to the first drive voltage is controlled to be first duration. It may be understood that the air valve operates with the first drive voltage for the first duration. The first duration may be preset, for example, several nanoseconds, several microseconds, several milliseconds, or several seconds. In this way, it is only necessary to increase the drive voltage of the air valve for a short period at the beginning of inflation to enhance the magnetic attraction for blocking the deflation hole, so that good air tightness of the entire blood pressure measurement system is ensured during an initial inflation stage with low air pressure.

Correspondingly, in an implementation, a processor may send a first instruction to a drive circuit 2, and the drive circuit 2 drives, in response to the first instruction, the air valve to start to operate.

During actual application, specific drive information, for example, the first drive voltage and the first duration, may be carried in the first instruction, or may be pre-burned into the drive circuit 2. In the former manner, the processor may flexibly modify the drive information each time when sending the first instruction, to conveniently adjust the drive information such as the first drive voltage or the first duration at any time, to adapt to different blood pressure measurement requirements. In the latter manner, because the drive information has been pre-burnt into the drive circuit 2, the processor only needs to send the first instruction that instructs the drive circuit 2 to start to operate, and does not need to carry additional drive information. This simplifies the first instruction, and speed up data transmission, thereby improving efficiency of blood pressure measurement.

S302: At a second moment of the pressurization phase, control the drive voltage of the air valve to gradually decrease from the first drive voltage to a second drive voltage.

The second moment may be understood as a moment after the first moment has elapsed for the first duration. The initial inflation stage is started from the second moment. As described above, the air pressure at the initial inflation stage is low, and the air valve only needs a small drive voltage to ensure that the deflation hole does not release air. Therefore, the processor may control, at the second moment of the pressurization phase, the drive voltage of the air valve to slowly decrease from the first drive voltage to the second drive voltage. Herein, the second drive voltage may be a minimum voltage in the drive voltage range that ensures the normal operation of the air valve. That is, the drive voltage range of the air valve is from the second drive voltage to the first drive voltage, and the air valve can function normally within the range.

In an implementation, the processor may preset second duration, and the second duration is used to limit a time during which the first drive voltage decreases to the second drive voltage. If the time during which the first drive voltage decreases to the second drive voltage is too long, that is, in the initial inflation stage, the voltage of the air valve remains high for a long time, an objective of reducing power consumption of the air valve cannot be achieved.

Correspondingly, in an implementation, the processor may send a second instruction to the drive circuit 2, and the drive circuit 2 decreases the drive voltage of the air valve in response to the second instruction.

During actual application, specific drive information, for example, the second drive voltage and the second duration, may be carried in the second instruction, or may be pre-burned into the drive circuit 2. For advantages of different manners, refer to the foregoing description. Details are not described herein again.

S303: At a third moment of the pressurization phase, control the drive voltage of the air valve to gradually increase from the second drive voltage to a third drive voltage.

The third moment may be understood as a moment after the second moment has elapsed for the second duration, or a moment at which the drive voltage of the air valve reaches the second drive voltage. A middle-to-later inflation stage is started from the third moment. The air bladder is gradually pressurized to highest air pressure, and expands to a final form. To ensure that no air is released, the drive voltage of the air valve may be gradually increased to enhance the magnetic attraction of the air valve, so as to block the deflation hole. Herein, the third drive voltage may be the first drive voltage, that is, the maximum voltage in the drive voltage range that ensures the normal operation of the air valve.

In an implementation, the processor may control, at a constant rate, the drive voltage of the air valve to gradually increase from the second drive voltage to the third drive voltage. In this way, a constant inflation rate of the air bladder may be matched. This prevents the air valve from being pressurized too quickly, which causes a waste of power consumption of the air valve, or too slowly, which causes the air valve to release air.

Correspondingly, in an implementation, the processor may send a third instruction to the drive circuit 2, and the drive circuit 2 increases the drive voltage of the air valve in response to the third instruction.

During actual application, specific drive information, for example, the third drive voltage and a pressurization rate of the air valve, may be carried in the third instruction, or may be pre-burned into the drive circuit 2. For advantages of different manners, refer to the foregoing description. Details are not described herein again.

According to the foregoing steps S301 to S303, the processor may control the air valve to operate at different moments of the pressurization phase by using different drive voltages. In this way, the drive voltage of the air valve is dynamically adjusted, so that the drive voltage of the air valve first decreases and then increases, thereby reducing a power loss of the air valve in the inflation process, and reducing overall power consumption of the blood pressure measurement system.

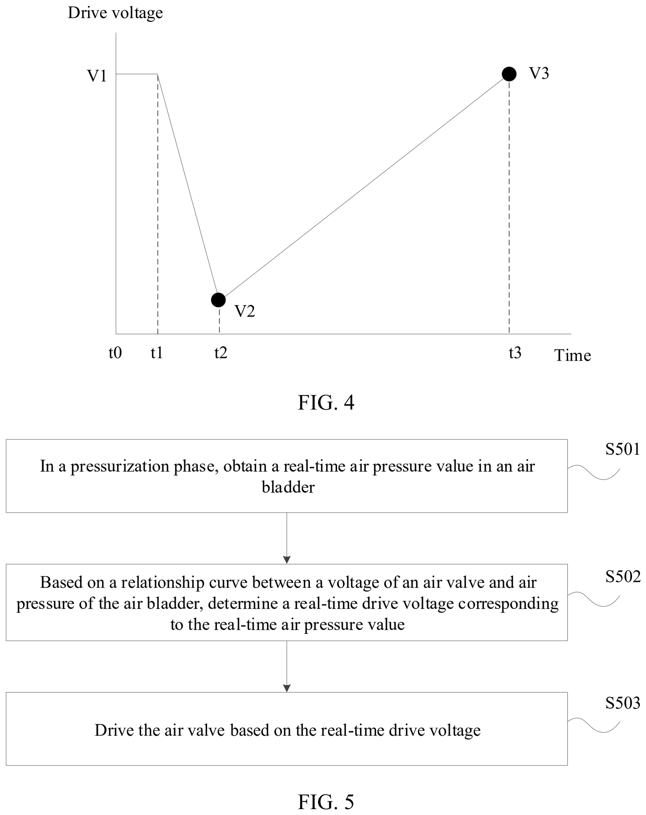

For example, FIG. 4 is a diagram of a drive voltage control curve according to an embodiment of this application. The curve shows changes of the drive voltage at different moments in the pressurization phase. During actual application, the curve is pre-stored into memory of the processor. In the pressurization phase, the processor directly reads the curve from the memory, so that the drive voltage of the air valve is controlled to be the first drive voltage (V1) at the first moment (t0); the drive voltage of the air valve is controlled to gradually decrease from V1 to the second drive voltage (V2) at the second moment (t1); and the drive voltage of the air valve is controlled to gradually increase from V2 to the third drive voltage (V3) at the third moment (t2). It should be noted again that the drive voltage control curve shown in FIG. 4 is merely an example. This is not limited in this application. For example, in another implementation, between t0 and t1, V1 may fluctuate up and down. A process in which V1 decreases to V2 may be a non-linear decrease. A process in which V2 increases to V3 may be a non-linear increase.

The following describes a second implementation of the blood pressure measurement method. In the second implementation, as mentioned above, the processor may dynamically adjust the drive voltage of the air valve in the pressurization process based on the real-time air pressure in the air bladder, to ensure that the air valve can complete operation with low power consumption.

FIG. 5 is a schematic flowchart of a second blood pressure measurement method according to an embodiment of this application. As shown in FIG. 5, the blood pressure measurement method may include the following steps.

S501: In a pressurization phase, obtain a real-time air pressure value in the air bladder.

Refer to FIG. 1. The sensor connected to the air bladder through the air path is configured to detect an air pressure value in the air bladder, so that the processor can obtain the real-time air pressure value in the air bladder by using the sensor.

In an implementation, the sensor detects air pressure in the air bladder in real time and then transmits the real-time detected air pressure value to the processor, and the processor receives the real-time air pressure value from the sensor. It may be understood that a higher detection frequency of the sensor indicates a more accurate real-time air pressure value obtained by the processor.

In another implementation, the sensor does not automatically send the air pressure value detected by the sensor to the processor, and the processor may obtain a current reading from the sensor as the real-time air pressure value when required.

Considering the objective of obtaining an accurate real-time air pressure value in time to dynamically adjust a voltage of the air valve in the pressurization phase, this application adopts the former implementation in which the sensor automatically sends the real-time detected air pressure value to the processor.

S502: Based on a relationship curve between a voltage of the air valve and air pressure of the air bladder, determine a real-time drive voltage corresponding to the real-time air pressure value.

In a related implementation, the relationship curve between the voltage of the air valve and the air pressure of the air bladder shown in FIG. 2 is used. The voltage of the air valve is fixed and remains high under different air pressure of the air bladder, which results in a waste of power of the air valve when the air bladder is at low pressure.

Therefore, this embodiment of this application defines a new correspondence between the voltage of the air valve and the air pressure of the air bladder. In the correspondence between the voltage of the air valve and the air pressure of the air bladder, as the air pressure of the air bladder increases, the voltage of the air valve first decreases and then increases. For a reason and an advantage of this trend, refer to the first implementation of the blood pressure measurement method. Details are not described herein again.

During actual application, the blood pressure measurement system may be calibrated in advance, and the relationship curve, between the voltage of the air valve and the air pressure of the air bladder, representing the correspondence between the voltage of the air valve and the air pressure of the air bladder may be obtained through curve fitting. In the relationship curve between the voltage of the air valve and the air pressure of the air bladder, voltages of the air valve that correspond to different air pressure of the air bladder are minimum voltages required to ensure that the air valve does not release air. In this case, in the pressurization phase, the processor may directly determine, from the relationship curve between the voltage of the air valve and the air pressure of the air bladder based on the real-time air pressure value, a voltage of the air valve corresponding to the real-time air pressure value as the real-time drive voltage.

In an implementation, the relationship curve between the voltage of the air valve and the air pressure of the air bladder may be stored in memory of the processor for quick reading by the processor.

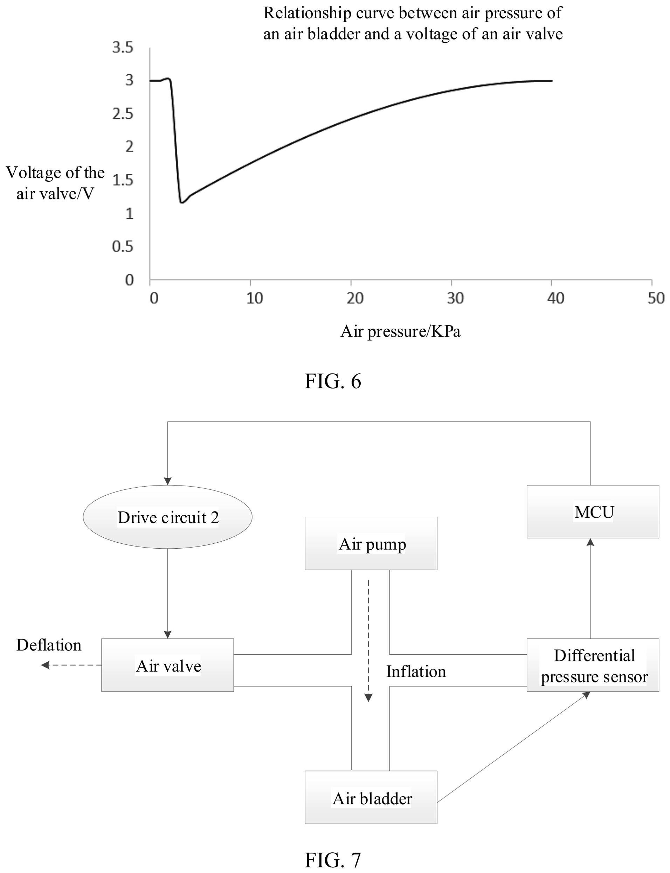

FIG. 6 is a diagram of a second relationship curve between air pressure of an air bladder and a voltage of an air valve according to an embodiment of this application. As shown in FIG. 6, in a process in which the air pressure of the air valve increases from 0 KPa to 40 KPa, the voltage of the air valve is first a high drive voltage (for example, 3 V), to ensure air tightness of the system at the beginning of inflation. Then, the voltage of the air valve gradually decreases from 3 V to about 1.2 V, to reduce a power loss of the air valve at an initial inflation stage. Subsequently, as the air pressure of the air bladder continues to increase, the voltage of the air valve gradually increases from 1.2 V to 3 V. That is, the drive voltage of the air valve is increased to enhance a magnetic attraction of the air valve, to avoid air release from the deflation hole. It should be noted that specific values in FIG. 6 are merely an example. This is not limited in this application.

In this case, after obtaining the real-time air pressure value, the processor may directly determine, from the relationship curve between the voltage of the air valve and the air pressure of the air bladder, the voltage of the air valve corresponding to the real-time air pressure value, and then use the corresponding voltage of the air valve as the real-time drive voltage. In this way, the processor can dynamically adjust the voltage of the air valve. As a result, in the pressurization process, the air valve is driven at a minimum voltage required to prevent the system from releasing air, thereby reducing a power loss of the air valve when the air bladder is at low pressure.

S503: Drive the air valve based on the real-time drive voltage.

Refer to FIG. 1. After determining the real-time drive voltage, the processor transmits the real-time drive voltage to the drive circuit 2. The drive circuit 2 adjusts the drive voltage of the air valve to the real-time drive voltage through the variable power supply control chip. In this way, the air valve is driven.

In an implementation, each time the processor receives a real-time air pressure value sent by the sensor, the processor determines a corresponding real-time drive voltage based on the relationship curve between the voltage of the air valve and the air pressure of the air bladder, and then drives the air valve based on the real-time drive voltage.

In another implementation, the processor stores the real-time air pressure value sent by the sensor. Specifically, the processor may store only a latest received real-time air pressure value, that is, overwrite a previously sent real-time air pressure value. Alternatively, the processor may store latest received real-time air pressure values in chronological order. When a quantity of the real-time air pressure values exceeds a specific quantity, the processor may then overwrite a previously sent real-time air pressure value. Regardless of the storage manner, the processor may read a latest received real-time air pressure value at a preset time interval, determine, based on the relationship curve between the voltage of the air valve and the air pressure of the air bladder, a real-time drive voltage corresponding to the latest received real-time air pressure value, and drive the air valve based on the real-time drive voltage. The preset time interval may be greater than or equal to a detection period of the sensor.

The following comprehensively describes the foregoing steps S501 to S503 in this application with reference to FIG. 7. FIG. 7 is a diagram of a blood pressure measurement control process according to an embodiment of this application. As shown in FIG. 7, a differential pressure sensor, an air bladder, an air valve, and an air pump are interconnected through an air path. First, the differential pressure sensor may obtain a real-time air pressure value in the air bladder by detecting air pressure in the air path, and then the differential pressure sensor transmits the real-time air pressure value to an MCU. After obtaining the real-time air pressure value, the MCU determines a corresponding real-time drive voltage based on a relationship curve between a voltage of the air valve and air pressure of the air bladder. The real-time drive voltage is a minimum voltage that ensures that a blood pressure measurement system does not release air under the corresponding real-time air pressure. Then, the MCU controls a drive circuit 2, so that the drive circuit 2 supplies power to the air valve with the real-time drive voltage through a variable power supply control chip of the drive circuit 2. In this way, the air valve can ensure good air tightness of the system by using a small power, thereby reducing overall power consumption of the blood pressure measurement system.

It may be understood that, in the second implementation, a first deflation condition mentioned in this application may be understood as a case in which the air valve does not release air, or a deflation amount of the air valve is less than or equal to a first deflation amount. The first deflation amount may be 0, or a value infinitely close to 0.

The following describes a third implementation of the blood pressure measurement method. In the third implementation, in the pressurization process, the processor may dynamically adjust both the drive voltage of the air valve and the drive voltage of the air pump based on real-time air pressure in the air bladder. This can not only ensure that the air valve can complete operation with low power consumption, but also ensure linear pressurization of the air bladder. The linear pressurization of the air bladder is a key to improving accuracy of blood pressure measurement.

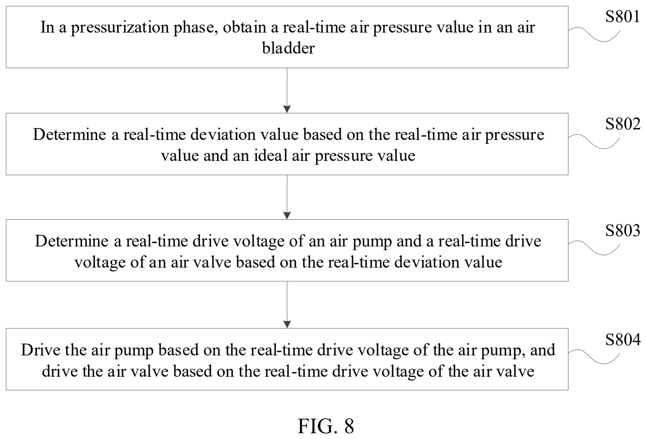

FIG. 8 is a schematic flowchart of a third blood pressure measurement method according to an embodiment of this application. As shown in FIG. 8, the blood pressure measurement method may include the following steps.

S801: In a pressurization phase, obtain a real-time air pressure value in the air bladder.

For this step, refer to the foregoing implementation of step S501. Details are not described herein again.

S802: Determine a real-time deviation value based on the real-time air pressure value and an ideal air pressure value.

The real-time deviation value may be an air pressure deviation value between the ideal air pressure value and the real-time air pressure value. The ideal air pressure value is a corresponding air pressure value at which the air bladder meets a linear pressurization condition. As described above, linear pressurization means that air pressure in the air bladder increases at a constant rate, or may be understood as that the air bladder inflates at a constant rate. In this way, the air bladder expands at a constant rate, so that a received blood pressure signal is more stable, thereby helping improve accuracy of blood pressure measurement. It may be understood that, in the pressurization phase, the ideal air pressure value of the air bladder increases linearly with time.

In an implementation, to enable the air bladder to meet the linear pressurization condition, a processor may store a linear pressurization curve of the air bladder. The linear pressurization curve includes ideal air pressure values corresponding to different moments. When determining the real-time deviation value, the processor first needs to determine, from the linear pressurization curve based on the current moment, an ideal air pressure value corresponding to the current moment, and then compares the ideal air pressure value with the real-time air pressure value, to obtain an air pressure deviation value between the ideal air pressure value and the real-time air pressure value, and the air pressure deviation value is used as the real-time deviation value.

S803: Determine a real-time drive voltage of the air pump and a real-time drive voltage of the air valve based on the real-time deviation value.

In this step, the processor may calculate the real-time drive voltage (hereinafter referred to as a fourth drive voltage) of the air pump and the real-time drive voltage (hereinafter referred to as a fifth drive voltage) of the air valve based on the real-time deviation value by using a proportional-integral-derivative (proportional-integral-derivative, PID) control algorithm. Under adjustment of the fourth drive voltage and the fifth drive voltage, the air pump inflates the air bladder with an inflation amount corresponding to the fourth drive voltage, and the air valve deflates the air bladder with a deflation amount corresponding to the fifth drive voltage, so that the air bladder meets the linear pressurization condition.

The deflation amount of the air valve is determined by a drive voltage of the air valve. A higher drive voltage of the air valve indicates a smaller deflation amount, and a lower drive voltage of the air valve indicates a larger deflation amount.

In an implementation, when calculating the fourth drive voltage and the fifth drive voltage based on the real-time deviation value by using the PID, the processor may further consider how to enable the air pump to be in an optimal operating state. The air pump operates in the optimal operating state, which can further improve inflating efficiency. An operating status of the air pump is adjusted by a drive circuit 1. The drive circuit 1 may adjust an operating parameter of the air pump, for example, a duty cycle or a drive voltage, to achieve the optimal operating state of the air pump. It should be noted that how to specify the operating parameter of the air pump to achieve the optimal operating state is determined based on an actual application scenario. The operating parameter, the optimal operating state, and a specific adjustment manner of the air pump are not limited in this application. For example, the air pump may achieve the optimal operating state when operating at a preset duty cycle. When calculating the fourth drive voltage and the fifth drive voltage based on the real-time deviation value by using the PID, the processor first considers the fourth drive voltage required for the air pump to be in the optimal operating state, and then considers, when the inflation amount is determined, the fifth drive voltage required for linear pressurization of the air bladder.

It may be understood that the real-time drive voltage of the air pump and the real-time drive voltage of the air valve are determined based on the real-time deviation value. This allows the air bladder to meet the linear pressurization condition, reduces power consumption of the air valve, and further ensures that the air pump operates in the optimal operating state, thereby improving inflating efficiency.

The following describes in detail how to calculate the real-time drive voltage of the air pump and the real-time drive voltage of the air valve by using the PID control algorithm. Details are not described herein.

In another implementation, in step S803, only the real-time drive voltage of the air valve may be adjusted based on the real-time deviation value, so that the air valve implements linear pressurization.

S804: Drive the air pump based on the real-time drive voltage of the air pump, and drive the air valve based on the real-time drive voltage of the air valve.

In this step, the processor transmits the real-time drive voltage of the air pump to the drive circuit 1, and then the drive circuit 1 adjusts a drive voltage of the air pump to the corresponding real-time drive voltage through a variable power supply control chip. In this way, the air pump is driven. Similarly, the processor transmits the real-time drive voltage of the air valve to the drive circuit 2, and then the drive circuit 2 adjusts a drive voltage of the air valve to the corresponding real-time drive voltage through the variable power supply control chip. In this way, the air valve is driven.

Compared with the second implementation of the blood pressure measurement method in which the air valve is closed in the pressurization phase to ensure that the system does not release air, however, in the third implementation of the blood pressure measurement method, the air valve is opened in the pressurization phase to release air. It is clear that, in the third implementation, the real-time drive voltage of the air valve is further reduced, thereby further reducing power consumption of the air valve.

It may be understood that, in the third implementation, the first deflation condition mentioned in this application may be understood as a case in which a deflation amount of the air valve is larger than a second deflation amount. The second deflation amount may be 0, or a value infinitely close to 0. An actual deflation amount of the air valve needs to allow the air bladder to meet the linear pressurization condition.

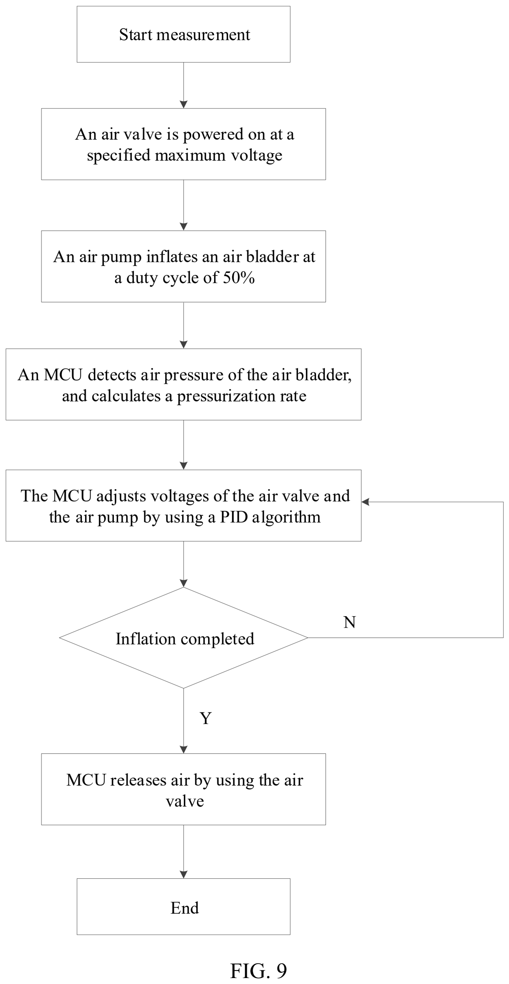

The following comprehensively describes a blood pressure measurement control process in steps S801 to S804 with reference to FIG. 9. As shown in FIG. 9, when blood pressure measurement starts, the air valve is controlled to be powered on at a specified maximum voltage to ensure air tightness of the system. Then, the air pump is controlled to inflate the air bladder at a duty cycle of 50% at a low voltage. After inflation starts, an MCU detects air pressure of the air bladder by using a differential pressure sensor, and calculates a pressurization rate. To control an inflation rate to be constant and ensure that the air pump operates in an optimal efficiency state, the MCU adjusts drive voltages of the air valve and the air pump by using the PID algorithm. Then, the processor determines whether the inflation is completed. If the inflation is not completed, return to a previous step, and the MCU continues to adjust the drive voltages of the air valve and the air pump. If the inflation is completed, the MCU may release air by using the air valve, or release air by using both the air valve and the air pump. It should be noted that the duty cycle of 50% is merely an example of an operating parameter of the air pump in the optimal operating state. This is not limited in this application.

The following describes in detail how the processor calculates the fourth drive voltage and the fifth drive voltage by using the PID control algorithm.

First, a basic concept of the PID control algorithm is briefly descried as follows: Based on a deviation value, a control value is calculated by using proportional, integral, and derivative terms. The control value is input to a controlled system. After receiving the input value, the system outputs a corresponding output value. A PID controller detects the output value and calculates a deviation. Then the preceding processes are repeated. The processor in this application may be the foregoing PID controller.

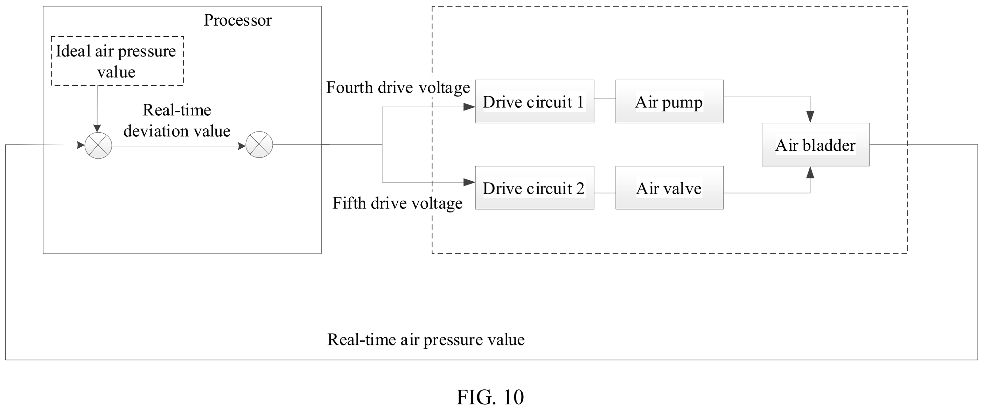

For ease of understanding, a specific process in which the processor adjusts the drive voltages of the air valve and the air pump by using the PID algorithm is described with reference to FIG. 10. As shown in FIG. 10, the processor detects a real-time air pressure value in the air bladder, and performs an operation on the real-time air pressure value and the ideal air pressure value, to obtain a real-time deviation value. Then, the processor calculates a control value based on the real-time deviation value by using proportional, integral, and derivative terms. Herein, the control value includes the fourth drive voltage and the fifth drive voltage, and a controlled system includes a drive circuit 1, an air pump, a drive circuit 2, an air valve, and an air bladder. The processor inputs the fourth drive voltage to the drive circuit 1, so that the drive circuit 1 drives the air pump based on the fourth drive voltage. In addition, the processor inputs the fifth drive voltage to the drive circuit 2, so that the drive circuit 2 drives the air valve based on the fifth drive voltage. In this way, when the air pump inflates the air bladder, the air valve also deflates the air bladder. By continuously obtaining the real-time air pressure value in the air bladder, the processor keeps adjusting the drive voltages of the air valve and the air pump, so that the air bladder is pressurized at a constant rate. In this way, in an entire inflating process, the air bladder implements linear pressurization, and the air pump keeps operating in an optimal state, so that overall power consumption of the blood pressure measurement system is reduced.

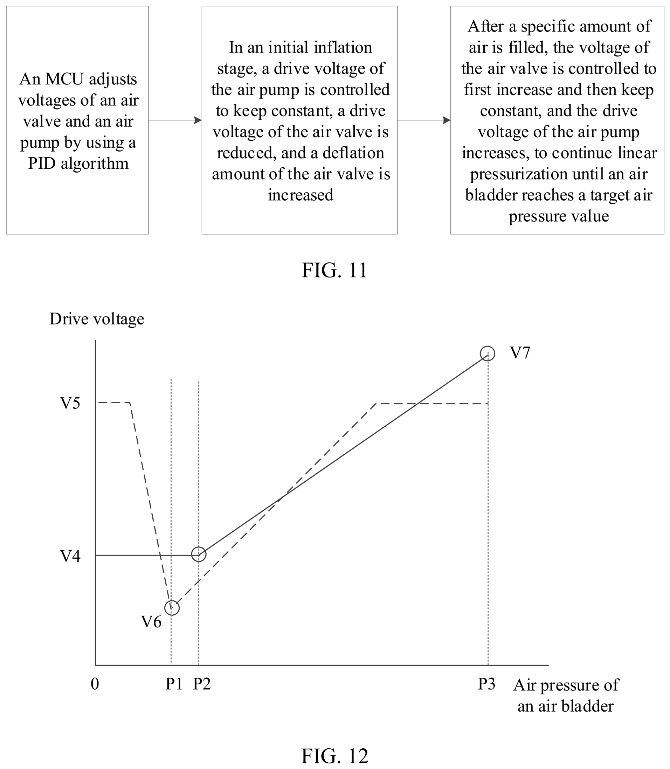

In an implementation, for a manner in which the MCU adjusts the drive voltages of the air valve and the air pump by using the PID algorithm, refer to FIG. 11. In an initial inflation stage, the MCU controls the drive voltage of the air pump to keep constant, reduces the drive voltage of the air valve, and increases a deflation amount of the air valve, to implement linear pressurization of the air bladder. After a specific amount of air is filled, that is, after the air bladder reaches specific air pressure, the MCU controls the drive voltage of the air valve to first increase and then keep constant, and increases the drive voltage of the air pump, to continue linear pressurization until the air bladder reaches a target air pressure value. FIG. 12 shows trends of the fourth drive voltage and the fifth drive voltage that change with air pressure of the air bladder after adjustment in the foregoing manner. A solid line represents a trend of the fourth drive voltage, and a dashed line represents a trend of the fifth drive voltage. As shown in FIG. 12, when inflation begins, that is, when the air pressure of the air bladder is 0, the fourth drive voltage is at a low position (V4), and the fifth drive voltage is at a high position (V5). In a process in which the air pressure of the air bladder gradually increases from 0 to P1, the fifth drive voltage gradually decreases from V5 to V6, to increase a deflation amount of the air valve, and the fourth drive voltage remains constant (V4). To ensure air tightness of the air bladder, a drive voltage of the air valve starts to gradually increase from P1, that is, the fifth drive voltage gradually increases from V6 to V5, and then keeps constant. When the air pressure of the air bladder reaches P2, a drive voltage of the air pump starts to increase, that is, the fourth drive voltage gradually increases from V4 to V7. In this case, the air bladder reaches a target air pressure value P3. It should be noted that a drive voltage change curve in FIG. 12 is merely an example, and the drive voltage change curve is not limited in this application. For example, in a process in which the fourth drive voltage increases from V4 to V7, the fourth drive voltage may increase linearly, or may increase nonlinearly. In a process in which the fifth drive voltage decreases from V5 to V6, the fifth drive voltage may decrease linearly, or may decrease nonlinearly.

It should be noted that a first drive circuit mentioned in this application may be the drive circuit 1 mentioned above, and a second drive circuit may be the drive circuit 2 mentioned above.

In addition, an embodiment of this application provides a blood pressure measurement system, including an air bladder, an air valve, and a blood pressure measurement apparatus. The blood pressure measurement apparatus is configured to perform any blood pressure measurement method in embodiments of this application.

It may be understood that as used in this specification, the term "module" may be or include an application-specific integrated circuit (ASIC), an electronic circuit, a memory and/or a processor (shared, dedicated, or a group) that executes one or more software or firmware programs, combined logic circuits, and/or another appropriate hardware component that provides a described function, or may be a part of these hardware components.

It may be understood that, in the implementations of this application, the processor may be a microprocessor (micro controller unit, MCU), a digital signal processor, a microcontroller, and/or any combination thereof. According to another aspect, the processor may be a single-core processor, a multi-core processor, and/or any combination thereof.

It should be noted that, in the examples and the specification of this patent, relational terms such as first and second are only used to distinguish one entity or operation from another entity or operation, and do not necessarily require or imply that any actual relationship or sequence exists between these entities or operations. Moreover, the terms "include", "contain", or any other variant thereof is intended to cover a non-exclusive inclusion, so that a process, a method, an article, or a device that includes a list of elements not only includes those elements but also includes other elements that are not expressly listed, or further includes elements inherent to such a process, method, article, or device. An element preceded by a statement "includes a/an" does not, without more constraints, preclude the presence of additional identical elements in the process, method, article, or device that includes the element.

Although this application has been illustrated and described with reference to some preferred embodiments of this application, a person of ordinary skill in the art should understand that various changes may be made to this application in terms of form and details without departing from the scope of this application.

## Claims

1. A blood pressure measurement method, applied to an electronic device, wherein the electronic device comprises an air bladder and an air valve that are configured for blood pressure measurement, and the method comprises:
when the electronic device enters a pressurization phase of blood pressure measurement, obtaining a real-time air pressure value in the air bladder;
determining a real-time drive voltage of the air valve based on the real-time air pressure value and a first deflation condition; and
driving, based on the real-time drive voltage, the air valve to operate.

2. The method according to claim 1, wherein determining the real-time drive voltage of the air valve based on the real-time air pressure value and the first deflation condition comprises:
when the first deflation condition is that a deflation amount of the air valve is less than or equal to a first deflation amount, obtaining a correspondence between a voltage of the air valve and air pressure of the air bladder;
determining, based on the correspondence between the voltage of the air valve and the air pressure of the air bladder, a voltage of the air valve corresponding to the real-time air pressure value; and
determining the voltage of the air valve corresponding to the real-time air pressure value as the real-time drive voltage of the air valve.

3. The method according to claim 2, wherein in the correspondence between the voltage of the air valve and the air pressure of the air bladder, as the air pressure of the air bladder increases, the voltage of the air valve exhibits a trend of first decreasing and then increasing.

4. The method according to claim 1, wherein determining the real-time drive voltage of the air valve based on the real-time air pressure value and the first deflation condition comprises:
when the first deflation condition is that a deflation amount of the air valve is greater than a second deflation amount, obtaining a real-time ideal air pressure value of the air bladder, wherein the real-time ideal air pressure value represents a corresponding air pressure value at which the air bladder meets a linear pressurization condition; and
determining the real-time drive voltage of the air valve based on a real-time deviation value between the real-time ideal air pressure value and the real-time air pressure value.

5. The method according to claim 4, wherein the electronic device further comprises an air pump configured for blood pressure measurement; and
determining the real-time drive voltage of the air valve based on the real-time deviation value between the real-time ideal air pressure value and the real-time air pressure value comprises:
determining a real-time drive voltage of the air pump and the real-time drive voltage of the air valve based on the real-time deviation value by using a proportional-integral-derivative control algorithm.

6. The method according to claim 5, wherein the electronic device further comprises a first drive circuit configured to drive the air pump to operate, and the method further comprises:
controlling, through a variable power supply control chip in the first drive circuit, a drive voltage of the air pump to be the real-time drive voltage of the air pump.

7. The method according to claim 4, wherein obtaining the real-time ideal air pressure value of the air bladder comprises:
obtaining a linear pressurization curve, wherein the linear pressurization curve comprises ideal air pressure values corresponding to different moments; and
determining, based on the linear pressurization curve, an ideal air pressure value corresponding to a current moment, to obtain the real-time ideal air pressure value.

8. The method according to claim 1, wherein the electronic device further comprises a sensor connected to the air bladder through an air path; and
obtaining the real-time air pressure value in the air bladder comprises:
detecting air pressure in the air bladder by using the sensor, to obtain the real-time air pressure value.

9. The method according to any one of claims 1 to 8, wherein the electronic device further comprises a second drive circuit configured to drive the air valve to operate; and
driving, based on the real-time drive voltage, the air valve to operate comprises:
controlling, through a variable power supply control chip in the second drive circuit, a drive voltage of the air valve to be the real-time drive voltage, so that the deflation amount of the air valve meets the first deflation condition.

10. A blood pressure measurement system, comprising an air bladder, an air valve, and a blood pressure measurement apparatus, wherein
the blood pressure measurement apparatus is configured to: in a pressurization phase of blood pressure measurement, obtain a real-time air pressure value in the air bladder; determine a real-time drive voltage of the air valve based on the real-time air pressure value and a first deflation condition; and drive, based on the real-time drive voltage, the air valve to operate.

11. A readable medium, wherein the readable medium stores instructions, and when the instructions are executed on an electronic device, the electronic device is enabled to perform the blood pressure measurement method according to any one of claims 1 to 9.

12. An electronic device, wherein the electronic device comprises:
a memory, configured to store instructions executed by one or more processors of the electronic device, and
a processor, wherein the processor is one of the processors of the electronic device, and is configured to perform the blood pressure measurement method according to any one of claims 1 to 9.
